# FASCICULE DE BREVET EUROPEEN

(11) **EP 1 093 787 B1**
(45) Date de publication et mention de la délivrance du brevet: **14.03.2007**
(21) Numéro de dépôt: 00402660.5
(22) Date de dépôt: 26.09.2000
(51) Int. Cl.: A61Q 5/02, A61Q 5/12, A61K 8/27, A61K 8/49, A61K 8/81, A61K 8/68, A61K 8/891

(54) **Composition de traitement antipelliculaire à base d'un sel de pyridinethione, d'un agent de conditionnement insoluble et d'un terpolymère acrylique**
Zusammensetzung zur Behandlung der Haarschuppen basiert auf einem Salz von Pyridinethione, einem unlöslichen Konditionierungsmittel und einem Acrylterpolymer
Composition for treating dandruff based on a pyridinethione salt, an insoluble conditioning agent and an acrylic terpolymer

(30) Priorité: 29.09.1999 FR 9912166
(43) Date de publication de la demande: 25.04.2001
(73) Titulaire: L'ORÉAL, 75008 Paris (FR)
(72) Inventeur: Maurin, Véronique, F-75016 Paris (FR); Beauquey, Bernard, F-92110 Clichy (FR)
(74) Mandataire: Casalonga, Axel

(56) Documents cités:
- EP-A- 0 824 914
- EP-A- 0 825 200
- EP-A- 0 878 186
- EP-A- 0 915 081
- US-A- 5 661 118

## Description

La présente invention concerne d'une manière générale des compositions de traitement antipelliculaire des cheveux et du cuir chevelu, à base d'un sel de pyridinethione, d'un agent de conditionnement insoluble et d'un terpolymère acrylique, ainsi qu'un procédé de traitement mettant en oeuvre ces compositions.

Les désordres desquamatifs du cuir chevelu tels que les pellicules sont liés à la présence d'une levure caractéristique appelée le Malassezia ovalis, levure anciennement dénommée Pityrosporum (P. ovale et P. orbiculare). Le traitement usuel des pellicules fait intervenir l'utilisation d'agents antipelliculaires, tel que des sels de pyridinethione dans un milieu, notamment un shampooing, un gel ou une lotion, qui est apte à répartir ces agents et à les déposer sur les téguments.

Il a été constaté que les traitements utilisant les sels de pyridinethione présentaient souvent l'inconvénient d'altérer les fibres kératiniques, et plus particulièrement les fibres kératiniques sensibilisées, en diminuant leurs performances cosmétiques, notamment en les rendant plus rêches et plus chargées.

Il existe donc un besoin d'une composition cosmétique antipelliculaire à base de sels de pyridinethione comme par exemple un shampooing, une lotion ou un gel, qui permette d'obtenir des performances cosmétiques acceptables, notamment au niveau de la douceur des cheveux.

La présente invention a pour objet des compositions de traitement antipelliculaire des cheveux et du cuir chevelu à base de sels de pyridinethione conférant aux cheveux les mêmes propriétés cosmétiques que celles obtenues lors de l'utilisation d'une composition antipelliculaire ne comprenant pas de sels de pyridinethione.

La demanderesse a découvert, de façon surprenante, qu'il était possible de formuler des compositions de traitement des matières kératiniques, notamment des lotions et shampooings, ayant les propriétés recherchées, en utilisant dans ces compositions des sels de pyridinethione et un agent de conditionnement insoluble associés à un terpolymère acrylique spécifique, défini ci-après. En effet, il a été constaté que l'utilisation des compositions de la présente invention permettait d'améliorer les propriétés cosmétiques des cheveux et du cuir chevelu, particulièrement en apportant plus de douceur et de souplesse aux cheveux sensibilisés mouillés et en apportant plus de douceur, de lissage aux cheveux séchés. L'utilisation des compositions de l'invention permet aussi d'obtenir une chevelure présentant au regard un aspect plus lisse.

Il a été également constaté que les associations de l'invention présentaient une bonne solubilité et une bonne tolérance cutanée et facilitaient le démêlage des cheveux séchés.

D'autres objets de l'invention apparaîtront à la lumière de la description et des exemples qui suivent.

Selon l'invention, les compositions de traitement antipelliculaire des cheveux et du cuir chevelu sont essentiellement caractérisées en ce qu'elles comprennent, dans un milieu cosmétiquement acceptable, au moins un sel de pyridinethione, au moins un agent de conditionnement insoluble et au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide;
- de 0,1 à 30% en poids, préférentiellement de 0,1 à 10% en poids d'un monomère (c) choisi parmi :
   un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
   un monomère tensioactif insaturé éthylénique copolymérisable obtenu par condensation d'un tensioactif non-ionique avec un acide carboxylique insaturé α,β-éthylénique ou son anhydride;
   un monomère tensioactif choisi parmi les produits de réaction de type urée d'un monoisocyanate insaturé monoéthylénique avec un tensioactif non-ionique présentant une fonction amine;
   un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy, n est égal à zéro ou désigne un nombre entier inférieur ou égal à 200 et préférentiellement inférieur ou égal à 100, m et p désignent zéro ou un nombre entier inférieur à n et R₂ est un groupe hydrophobe d'au moins 8 atomes de carbone et préférentiellement en C₈-C₃₀; et
   un monomère non-ionique de type uréthane produit par réaction d'un tensioactif non ionique monohydrique avec un isocyanate insaturé monoéthylénique;
- et éventuellement au moins un monomère de réticulation;
les pourcentages en poids de monomères étant basés sur le poids total des monomères constituant le terpolymère.

Selon un mode de réalisation de l'invention, le terpolymère acrylique est présent à raison de 0,01 à 20 % en poids de matière active (M.A.), de préférence 0,1 à 10 % en poids par rapport au poids total de la composition.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropyl-acrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé α,β-éthylénique ou son anhydride, de préférence les acides mono ou dicarboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion par exemple. Des terpolymères conformes à l'invention et leurs méthodes de préparation sont notamment décrits dans les demandes EP-A-0824914 et EP-A-0825200.

Parmi ces terpolymères, on préfère utiliser en particulier le polymère "STRUCTURE^{®} PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A..

En plus de ces monomères, le terpolymère peut contenir d'autres monomères qui permettent de réticuler ledit terpolymère. Ces monomères sont utilisés dans des proportions assez faibles, jusqu'à 2% en poids par rapport au poids total des monomères utilisés pour préparer le terpolymère. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusièurs substituants vinyle tels que des diènes, triènes et tétraènes. Des monomères de réticulation peuvent notamment être des divinyl-benzènes, des trivinyl-benzènes, le 1,2,4-trivinylcyclohexène, le 1,5-hexadiène, le 1,5,9-décatriène, le 1,9-décadiène, le 1,5-heptadiène, des di-allyl phthalates, de l'éthylène glycol diméthacrylate, des polyéthylène glycol diméthacrylates, des penta- et tétra-acrylates, des triallyl pentaérythritols, des octaallyl saccharoses, des cycloparaffines, des cyclooléfines et du N-méthylène-bis-acrylamide.

Les sels de pyridinethione sont notamment les sels de calcium, de magnésium, de baryum, de strontium, de zinc, de cadmium, d'étain et de zirconium. Le sel de zinc de pyridinethione est particulièrement préféré.

Le sel de zinc de pyridinethione est notamment commercialisé sous la dénomination Omadine de zinc par la société ARCH CHEMICALS.

Les sels de pyridinethione sont présents dans des proportions notamment comprises entre 0,001% et 10% en poids par rapport au poids total de la composition et préférentiellement dans des proportions comprises entre 0,01% et 5% en poids par rapport au poids total de la composition.

Les compositions selon l'invention contiennent de préférence en outre au moins un agent tensio-actif, notamment choisi parmi les tensio-actifs anioniques, amphotères, non-ioniques, cationiques et leurs mélanges.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates et les N-acyltaurates.

Le radical alkyle ou acyle de ces différents composés est généralement constitué par une chaîne carbonée comportant de 8 à 30 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 30 atomes de carbone.

On peut également utiliser des agents tensio-actifs considérés comme faiblement anioniques tels que les acides alkyl ou alkylaryl éthers carboxyliques polyoxyalkylénés ou leurs sels, les acides alkylamido éthers carboxyliques polyoxyalkylénés ou leurs sels, les acides d'alkyl D-galactoside uroniques ou leurs sels.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 30 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensio-actifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproamphodipropionate, Disodium Capryloamphodipropionate, Lauroamphodipropionate acide, Cocoamphodipropionate acide.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par ex. chlorures) de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyldiméthyl (myristyl acétate) ammonium commercialisé sous la dénomination «CEPHARYL 70» par la société VAN DYK.

On peut également utiliser les sels (chlorures ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

De préférence, on utilise au moins un tensioactif détergent, notamment si l'on désire obtenir des shampooings antipelliculaires.

Les agents tensio-actifs sont éventuellement utilisés dans les compositions conformes à l'invention dans des proportions de 0,01 à 50% en poids par rapport au poids total de la composition. Quand les compositions sont sous forme de shampooings, ils sont généralement utilisés à raison d'au moins 4% en poids, de préférence entre 5 et 50% en poids par rapport au poids total de la composition, et en particulier entre 8 et 35%.

L'agent de conditionnement insoluble peut être défini comme un agent insoluble en solution aqueuse permettant d'améliorer au moins une propriété cosmétique de la fibre capillaire telle que par exemple le démêlage, la douceur, le lissage ou le volume.

Il est préférentiellement choisi parmi:

### A. Les céramides :

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131.

Les composés de type céramide utilisables selon la présente invention répondent préférentiellement à la formule générale (I): 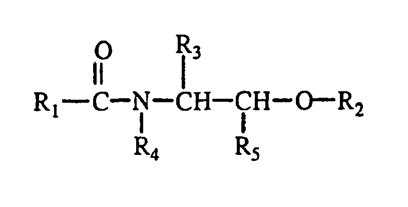 dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié(s) par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié(s) par un acide gras saturé ou insaturé, linéaire ou ramifié, éventuellement mono ou polyhydroxylé, en C₁-C₃₅;
   - soit un radical R"-(NR-CO)-R', dans lequel R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
   - soit un radical R₈-O-CO-(CH₂)p, R₈ désignant un radical hydrocarboné en C₁-C₂₀, p étant un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
   de préférence, R₃ désigne un radical α-hydroxyalkyle saturé ou insaturé en C₁₂-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical Rg-O-CO-(CH₂)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)n, (galactosyle)m, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle, R₄ ne désigne pas un atome d'hydrogène, un radical méthyle ou éthyle.

Parmi les composés de formule (I), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179.

Les composés de type céramide plus particulièrement préférés selon l'invention sont les composés de formule (I) pour lesquels R₁ désigne un alkyle saturé ou insaturé dérivé d'acides gras en C₁₄-C₂₂ éventuellement hydroxylé; R₂ désigne un atome d'hydrogène; et R₃ désigne un radical alkyle saturé ou insaturé linéaire en C₁₂-C₁₈ éventuellement hydroxylé et de préférence désigne un radical α-hydroxyalkyle saturé ou insaturé en C₁₆.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) dans lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994, EP-A-0 647 617, EP-A-0 736 522 et WO 94/07844.

De tels composés sont par exemple le QUESTAMIDE H (bis-(N-hydroxyéthyl N-cétyl) malonamide) vendu par la société QUEST et le N-(2-hydroxyéthyl)-N-(3-cétyloxy-2-hydroxypropyl)amide d'acide cétylique.

On peut également utiliser le N-docosanoyl N-méthyl-D-glucamine décrit dans la demande de brevet WO94/24097.
Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine ou les mélanges de ces composés.

### B. Les cires:

Conformément à l'invention, elles sont choisies parmi les cires animàles, les cires végétales, les cires minérales et les cires synthétiques. Les cires peuvent être fluorées ou perfluorées.

Elles sont notamment choisies parmi la cire de Candellila, la cire de Carnauba, la cire de tournesol, la cire d'Ouricuri, la cire de canne à sucre, les cires de polyéthylène, la cire d'abeilles, les cires de lanoline, les cires de paraffine, les cires de cérasine, les cires microcristallines, les ozokérites, les spermaceti, et les cires de jojoba hydrogénées.

### C. Les silicones insolubles :

Dans le cadre de la présente invention, les silicones insolubles sont des polyorganosiloxanes modifiés ou non, à savoir des huiles de polyorganosiloxanes ou des gommes ou des résines de polyorganosiloxanes, telles quelles ou sous forme de solutions dans des solvants organiques ou encore sous forme d'émulsions ou de microémulsions.

Parmi les polyorganosiloxanes pouvant être utilisés conformément à la présente invention, on peut citer à titre non limitatif :
I. Les silicones volatiles : celles-ci possèdent un point d'ébullition compris entre 60°C et 260°C. Elles sont choisies parmi les silicones cycliques contenant de 3 à 7 atomes de silicium et de préférence 4 à 5. Il s'agit, par exemple, de l'octaméthyl-cyclotétrasiloxane vendu sous le nom de "VOLATILE SILICONE 7207" par UNION CARBIDE ou "SILBIONE 70045 V2" par RHONE POULENC, le décaméthylcyclopentasiloxane vendu sous le nom de "VOLATILE SILICONE 7158" par UNION CARBIDE, "SILBIONE 70045 V5 par RHONE POULENC, ainsi que leurs mélanges. On cite également les cyclocopolymères du type diméthylsiloxane/méthylalkylsiloxane, tel que la "SILICONE VOLATILE FZ 3109" vendue par la Société UNION CARBIDE, qui est un cyclocopolymère diméthylsiloxane/méthyloctylsiloxane.
II. Les silicones non volatiles : elles sont constituées principalement par:
   (i) les polyalkylsiloxanes ; Parmi les polyalkylsiloxanes, on peut citer principalement les polydiméthylsiloxanes linéaires à groupements terminaux triméthylsilyle, comme par exemple, et à titre non limitatif, les huiles "SILBIONE" de la série 70047 commercialisées par RHODIA CHIMIE;
   (ii) les polyarylsiloxanes ;
   (iii) les polyalkylarylsiloxanes ; on peut citer les polyméthylphénylsiloxanes, les polydiméthylméthylphénylsiloxanes, les polydiméthyldiphénylsiloxanes linéaires et ramifiés, tels que par exemple l'huile "RHODORSIL 763" de RHODIA CHIMIE;
   (iv) les gommes de silicone ; ce sont des polydiorganosiloxanes de masse moléculaire comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges; on cite, par exemple, les composés suivants :
      - polydiméthylsiloxane,
      - poly[(diméthylsiloxane)/(méthylvinylsiloxane)],
      - poly[(diméthylsiloxane)/(diphénylsiloxane)],
      - poly[(diméthylsiloxane))(phénylméthylsiloxane)],
      - poly[(diméthylsiloxane)/(diphénylsiloxane)/(méthylvinylsiloxane)];
      on peut aussi citer, par exemple, à titre non limitatif, les mélanges suivants :
      1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401" vendu par la Société DOW CORNING;
      2) les mélanges formés à partir d'une gomme polydiméthylsiloxane avec une silicone cyclique, tel que le produit "SF 1214 SILICONE FLUID" de GENERAL ELECTRIC, qui est une gomme SE 30 de PM 500 000 solubilisée dans la "SF 1202 SILICONE FLUID" (décaméthylcyclopentasiloxane);
      3) les mélanges de deux PDMS de viscosité différente, notamment d'une gomme PDMS et d'une huile PDMS, tels que les produits "SF 1236" et "CF 1241" de la Société GENERAL ELECTRIC;
   (v) les résines de silicone ; de préférence des systèmes siloxaniques réticulés renfermant les unités R₂SiO_{2/2}, RSiO_{3/2} et SiO_{4/2} dans lesquelles R représente un groupement hydrocarboné possédant 1 à 6 atomes de carbone ou un groupement phényle. Parmi ces résines, on peut citer le produit vendu sous la dénomination "DOW CORNING 593";
   (vi) les polyorganosiloxanes organomodifiés ; c'est-à-dire des silicones telles que définies précédemment, comportant dans leur structure générale, un ou plusieurs groupements organofonctionnels directement fixés sur la chaîne siloxanique ou fixés par l'intermédiaire d'un radical hydrocarboné; on cite, par exemple, les silicones comportant :
      a) des groupements polyéthylèneoxy et/ou polypropylèneoxy, comportant éventuellement des groupes alkyle, tels que le produit dénommé diméthicone copolyol vendu par la Société DOW CORNING sous la dénomination "DC 1248", et l'alkyl (C12) méthicone copolyol vendu par la Société DOW CORNING sous la dénomination "Q2 5200";
      b) des groupements (per)fluorés comme les groupements trifluoroalkyle, telles que, par exemple, celles vendues par la Société GENERAL ELECTRIC sous les dénominations "FF.150 Fluorosilicone Fluid";
      c) des groupements hydroxyacylamino, telles que celles décrites dans la demande de brevet européen EP-A-0 342 834 et en particulier la silicone vendue par la Société DOW CORNING sous la dénomination "Q2-8413" ;
      d) des groupements thiols comme dans les silicones "X 2-8360" de DOW CORNING ou les "GP 72A" et "GP 71" de GENESEE ;
      e) des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ou aminoalkyl(C₁-C₄)aminoalkyl(C₁-C₄). On utilise plus particulièrement les silicones dénommées amodiméthicone et triméthylsilylamodiméthicone selon la dénomination CTFA (1997);
      f) des groupements carboxylates, comme les produits décrits dans le brevet européen EP 186 507 de CHISSO CORPORATION;
      g) des groupements hydroxylés, comme les polyorganosiloxanes à fonction hydroxyalkyle, décrits dans la demande de brevet FR-A-2 589 476 ;
      h) des groupements alcoxylés comportant au moins 12 atomes de carbone comme le produit "SILICONE COPOLYMER F 755" de SWS SILICONES;
      i) des groupements acyloxyalkyle comportant au moins 12 atomes de carbone, comme par exemple les polyorganosiloxanes décrits dans la demande de brevet FR-A-2 641 185;
      j) des groupements ammonium quaternaire, comme dans le produit "ABIL K 3474" de la Société GOLDSCHMIDT ;
      k) des groupements amphotères ou bétaïniques, tels que dans le produit vendu par la Société GOLDSCHMIDT sous la dénomination "ABIL B 9950" ;
      l) des groupements bisulfite, tels que dans les produits vendus par la Société GOLDSCHMIDT sous les dénominations "ABIL S 201 " et "ABIL S 255";
   (vii) les copolymères blocs ayant un bloc linéaire polysiloxane-polyalkylène comme unité répétitive ; la préparation de tels copolymères blocs mis en oeuvre dans le cadre de la présente invention est décrite dans la demande européenne EP 0 492 657 A1;
   (viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; notamment ceux choisis plus préférentiellement parmi ceux décrits dans les brevets US 4.963.935, US 4.728.571 et US 4.972.037 et les demandes de brevet EP-A 0 412 704, EP-A-0 412 707, EP-A-0 640 105 et WO 95/00578;
   (ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; des exemples de tels polymères, ainsi que leur mode particulier de préparation, sont notamment décrits dans les demandes de brevets EP-A-0 582 152, WO 93/23009 et WO 95/03776;
   (x) ou leurs mélanges.

Les polyorganosiloxanes préférés pour être utilisés selon l'invention sont les polyorganopolysiloxanes non volatils et préférentiellement les huiles ou gommes polydiméthylsiloxanes aminés, arylés ou alkylarylés.

### D. Les huiles non siliconées:

Les huiles non siliconées utilisées conformément à l'invention, sont des huiles de synthèse, des huiles minérales, végétales, de préférence naturelles, ou animales, des alcools gras insaturés, des esters d'acides gras et de mono-ou polyalcools inférieurs en C₂-C₄.

Les huiles de synthèse sont notamment les polyoléfines en particulier les poly-α-oléfines et plus particulièrement:
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 150000.
   A titre d'exemples de poly- α-oléfines utilisables dans le cadre de la présente invention, on peut plus particulièrement mentionner les polyisobutènes répondant à la formule (II) : 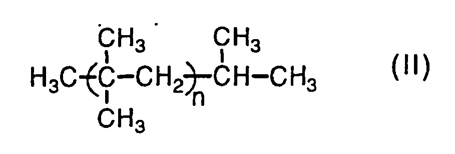 vendus sous le nom de PERMETHYL 99 A, 101A, 102 A, 104 A (n=16) par la Société PRESPERSE Inc, ou bien encore les produits vendus sous le nom de ARLAMOL HD (n=3) par la Société ICI (n désignant le degré de polymérisation),
   et ceux répondant à la formule(III) : 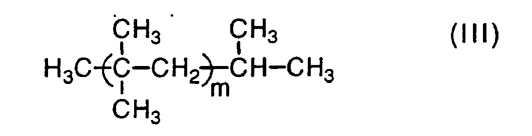 où m est supérieur ou égal à 18 et compris de préférence entre 18 et 40. Parmi les composés de formule (III), on peut citer le produit vendu sous la dénomination PERMETHYL 106A, dans lequel m est égal à 38.
- de type polydécène, hydrogéné ou non.

De tels produits sont vendus par exemple sous les dénominations ETHYLFLO par la société ETHYL CORP., et d'ARLAMOL PAO par la société ICI.

Une autre classe d'huiles de synthèse est constituée par les mono, di et triglycérides d'acides gras en C₆-C₂₂.

Les huiles minérales pouvant être utilisées sont choisies de préférence parmi les hydrocarbures, tels que l'hexadécane et l'huile de paraffine ou l'huile de vaseline.

On peut également utiliser les huiles essentielles naturelles telles que, par exemple, les huiles d'eucalyptus, de lavandin, de lavande, de menthe, de vétiver, de litsea cubeba,de citron, de santal, de romarin, de camomille, de sarriette, de noix de muscade, de cannelle, d'hysope, de carbi, d'orange, de géraniol, de cade et de bergamote.

Les huiles animales peuvent être choisies parmi les huiles naturellement ou chimiquement saturées telles que le squalane, l'huile de baleine, de phoque, de menhaden, de foie de flétan, de foie de morue, de thon, de suif, de boef, de cheval, de mouton, de vison et de loutre.

Les huiles précitées peuvent être utilisées en mélange dans les compositions conformes à l'invention.

L'agent de conditionnement est utilisé dans les proportions connues de l'homme du métier. Ainsi, il peut être présent dans des proportions de 0,001% à 60% en poids, et de préférence de 0,01 à 40% en poids par rapport au poids total de la composition.

Les céramides sont plus particulièrement utilisées dans des proportions de 0,005% et 5% en poids, et de préférence entre 0,01 et 3 % en poids par rapport au poids total de la composition.

Les cires sont préférentiellement utilisées dans des proportions de 0,1 à 20% et plus préférentiellement de 0,5 à 10% en poids par rapport au poids total de la composition.

Les polyorganosiloxanes sont préférentiellement utilisés dans les compositions de l'invention dans des proportions comprises entre 0,01 et 20% en poids et encore plus préférentiellement entre 0,1 et 10% en poids par rapport au poids total de la composition.

Les huiles non siliconées sont notamment utilisées dans des proportions de 0,01% à 20% et préférentiellement de 0,5% à 10% en poids par rapport au poids total de la composition.

Les compositions, selon l'invention, présentent un pH généralement compris entre 3 et 12, et plus particulièrement entre 4 et 8.

Le milieu cosmétiquement acceptable des compositions est constitué par de l'eau ou par un ou plusieurs solvants ou par un mélange d'eau et d'au moins un solvant cosmétiquement acceptable choisi parmi les alcools inférieurs, les alkylèneglycols et les éthers de polyol.

Dans un autre mode de réalisation préféré, les compositions de l'invention contiennent en outre au moins un polymère cationique choisi parmi tous ceux déjà connus en soi, notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques utilisés ont généralement une masse moléculaire comprise entre 500 et 5.10⁶ environ, et de préférence comprise entre 10³ et 3.10⁶ environ .

Parmi les polymères cationiques, on peut citer les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits dénommés dans le dictionnaire CTFA "TRIETHONIUM HYDROLYZED COLLAGEN ETHOSULFATE" ;
- les hydrolysats de collagène portant des groupements chlorure de triméthylammonium et de triméthylstéarylammonium, dénommés dans le dictionnaire CTFA "STEARTRIMONIUM HYDROLYZED COLLAGEN" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres le "CROQUAT L", le "CROQUAT M", le "CROQUAT S" et le "CROTEIN Q" vendus par la Société CRODA.

D'autre protéines ou hydrolysats quaternisés sont par exemple ceux vendus par la Société INOLEX, sous la dénomination "LEXEIN QX 3000".

On peut encore citer les protéines végétales quaternisées telles que les protéines de blé, de maïs ou de soja: comme protéines de blé quaternisées, on peut citer celles appelées dans le dictionnaire CTFA "COCODIMONIUM HYDROLYSED WHEAT PROTEIN", "LAURIDIMONIUM HYDROLYSED WHEAT PROTEIN", ou encore "STEARDIMONIUM HYDROLYSED WHEAT PROTEIN".

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer:
(1) Les copolymères vinylpyrrolidone-acrylate ou -méthacrylate de dialkylaminoalkyle quaternisés ou non, tels que les polymères décrits en détail dans les brevets français 2 077 143 et 2 393 573.
(2) Les dérivés d'éthers de cellulose comportant des groupements ammonium quaternaires décrits dans le brevet français 1 492 597.
(3) Les dérivés de cellulose cationiques tels que les copolymères de cellulose ou les dérivés de cellulose greffés avec un monomère hydrosoluble d'ammonium quaternaire, et décrits notamment dans le brevet US 4131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylamidopropyl triméthyl ammonium ou de diméthyl-diallylammonium.
(4) Les polysaccharides et notamment gommes de guar cationiques décrits plus particulièrement dans les brevets US 3 589 578 et 4 031 307.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé, l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, le rapport molaire entre la polyalkylène polyamine et l'acide dicarboxylique étant compris entre 0,8 : 1 et 1,4 : 1, le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(9) Les cyclopolymères de méthyl diallyl amine ou de diméthyl diallyl ammonium, notamment ceux décrits dans le brevet français 2.080.759 et dans son certificat d'addition 2.190.406.
(10) Les polymères de diammonium quaternaire décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) Les polymères de polyammonium quaternaire notamment décrits dans la demande de brevet EP-A-122 324.
(12) Les homopolymères ou copolymères dérivés des acides acrylique ou méthacrylique et comportant des motifs CH₂-CHRₐ-CO-O-A₁-NRₑR_{f} , CH₂-CHRₐ-CO-O-A₁-N⁺R_{b}R_{c}R_{d}, X⁻ et/ou CH₂-CHRₐ-CO-NH-A₁-N⁺R_{b}R_{c}R_{d}, X⁻
   dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
(13) Les polymères quaternaires de vinylpyrrolidone et de vinylimidazole tels que par exemple les produits commercialisés sous les dénominations "LUVIQUAT FC 905", "LUVIQUAT FC 550" et "LUVIQUAT FC 370" par la société BASF.
(14) Les polyamines comme le "POLYQUART H" vendu par HENKEL, référencé sous le nom de « POLYETHYLENEGLYCOL TALLOW POLYAMINE » dans le dictionnaire CTFA.
(15) Les polymères réticulés de sels de méthacryloyloxyéthyl triméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléhnique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère les dérivés d'éther de cellulose comportant des groupements ammonium quaternaires, les polysaccharides et notamment gomme de guar cationiques et les cyclopolymères de méthyl diallyl amine ou de diméthyl diallyl ammonium.

Les polymères cationiques sont utilisés dans les compositions de l'invention dans des proportions comprises entre 0,001 et 20% en poids et de préférence entre 0,05 et 5% en poids par rapport au poids total de la composition.

Les compositions selon l'invention peuvent également contenir en outre au moins un adjuvant choisi parmi les adjuvants habituellement utilisés en cosmétique, tel que les parfums, les conservateurs, les séquestrants, les humectants, les sucres, les polymères amphotères, le menthol, les dérivés de nicotinate, les agents anti-chute des cheveux, les stabilisateurs de mousse, les agents propulseurs, les colorants, les vitamines ou provitamines, les agents acidifiants ou alcalinisants ou d'autres adjuvants cosmétiques bien connus.

Dans une forme de réalisation préférée de l'invention, les compositions selon l'invention sont utilisées comme shampooings pour le lavage et le traitement des cheveux et du cuir chevelu. Elles peuvent aussi être utilisées comme lotions antipelliculaires ou après-shampooing antipelliculaires.

Le procédé de traitement des cheveux consiste à appliquer une composition telle que définie ci-dessus sur des cheveux humides ou secs dans des quantités efficaces, cette application étant suivie facultativement d'un rinçage après un temps de pose facultatif.

Les exemples qui suivent sont destinés à illustrer l'invention.

### EXEMPLE I : SHAMPOOING ANTIPELLICULAIRE

| | | |
|---|---|---|
| Distéarate de glycol | 2 | g |
| N-cocoyl amidoéthyl, N-éthoxycarboxyméthyl | | |
| glycinate de sodium à 38% M.A. vendu sous la | | |
| dénomination MIRANOL C2M CONC par | | |
| la société RHODIA CHIMIE | 2 | g |
| Cocoyl bétaïne en solution aqueuse à 30% | | |
| vendue sous la dénomination DEHYTON | | |
| AB 30 par la société HENKEL | 4 | g |
| Pyrithione de zinc en suspension aqueuse à 48% | 2 | g |
| Hydroxyéthyl cellulose réticulée à | | |
| l'épichlorhydrine, quaternisée par la triméthylamine | | |
| vendue sous la dénomination JR400 par la société | | |
| UNION CARBIDE CORPORATION | 0,25 | g |
| N-oléyl dihydrosphingosine | 0,1 | g |
| Lauryl éther sulfate de sodium (2,2 OE) | | |
| à 70% de M.A. | 18 | g |
| Terpolymère d'acrylates, d'amino(méth)acrylates | | |
| et d'itaconate d'alkyle en C₁₀-C₃₀ polyoxyéthyléné | | |
| à 20 moles d'oxyde d'éthylène en dispersion aqueuse | | |
| à 20% M.A. vendus sous la dénomination | | |
| "STRUCTURE^{®} PLUS" par la Société National Starch | 1,5 | g |
| Acide citrique | 3 | g |
| Parfum, conservateur | qs | |
| Eau déminéralisée stérilisée | qsp 100 | g |

On ajuste le pH à 5,5 par de l'hydroxyde de sodium.

Après utilisation de ce shampooing, les cheveux sensibilisés mouillés sont doux et les cheveux séchés sont doux et lisses au toucher et se démêlent facilement.
L'aspect général de la coiffure est lisse.

### EXEMPLE II : SHAMPOOING ANTIPELLICULAIRE

| | | |
|---|---|---|
| Propylène glycol | 0,1 | g |
| Cocoyl betaïne en solution aqueuse à 30% | 6 | g |
| Pyrithione de zinc en suspension aqueuse à 48% | 2 | g |
| Hydroxyéthyl cellulose réticulée à l'épichlorhydrine, | | |
| quaternisée par la triméthylamine vendue sous la | | |
| dénomination JR 400 par la société | | |
| UNION CARBIDE CORPORATION | 0,4 | g |
| Polydiméthylsiloxane commercialisé sous la | | |
| dénomination "MIRASIL DM 500.000" par la | | |
| société RHODIA CHIMIE | 1,5 | g |
| Mélange 1-(hexadécyloxy)-2-octadécanol/ | | |
| alcool cétylique | 2,5 | g |
| Monoisopropanolamide d'acides de coprah | 0,5 | g |
| Lauryl éther sulfate de sodium (2,2 OE) | | |
| à 70% de M.A. | 16,8 | g |
| Terpolymère d'acrylates, d'amino(méth)acrylates | | |
| et d'itaconate d'alkyle en C₁₀-C₃₀ polyoxyéthyléné | | |
| à 20 moles d'oxyde d'éthylène en dispersion aqueuse | | |
| à 20% M.A. vendus sous la dénomination | | |
| "STRUCTURE^{®} PLUS" par la Société National Starch | 1,5 | g |
| Parfums, Conservateurs | qs | |
| Eau déminéralisée stérilisée | qsp 100 | g |

On ajuste le pH à 6,5 par de l'hydroxyde de sodium.

Après utilisation de ce shampooing, les cheveux sensibilisés mouillés sont doux et les cheveux séchés sont doux et lisses au toucher et se démêlent facilement.
L'aspect général de la coiffure est lisse.

## Revendications

1. Composition de traitement antipelliculaire des cheveux et du cuir chevelu, comprenant dans un milieu cosmétiquement acceptable au moins un sel de pyridinethione et au moins un agent de conditionnement insoluble, **caractérisée par le fait qu'**elle comprend en outre au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di (C₁-C₄)alkylamino (C₁-C₄) alkyl (méth)acrylamide;
- de 0,1 à 30% en poids, préférentiellement de 0,1 à 10% en poids d'un monomère (c) choisi parmi :
un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
un monomère tensioactif insaturé éthylénique copolymérisable obtenu par condensation d'un tensioactif non-ionique avec un acide carboxylique insaturé α,β-éthylénique ou son anhydride;
un monomère tensioactif choisi parmi les produits de réaction de type urée d'un monoisocyanate insaturé monoéthylénique avec un tensioactif non ionique présentant une fonctionalité amine;
un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy, n est égal à zéro ou désigne un nombre entier inférieur ou égal à 200, m et p désignent zéro ou un nombre entier inférieur à n et R₂ est un groupe hydrophobe d'au moins 8 atomes de carbone; et un monomère non-ionique de type uréthane produit par réaction d'un tensioactif non ionique monohydrique avec un isocyanate insaturé monoéthylénique;
- éventuellement au moins un monomère de réticulation;
les pourcentages en poids de monomères étant basés sur le poids total des monomères constituant le terpolymère

2. Composition selon la revendication 1, **caractérisée en ce que** le terpolymère est présent à raison de 0,01 à 20% en poids de matière active, de préférence 0,1 à 10 % en poids par rapport au poids total de la composition.

3. Composition selon la revendication 1 ou 2, **caractérisée en ce que** le monomère (a) est choisi parmi les acrylates d'alkyle en C₂-C₆, et est préférentiellement l'acrylate d'éthyle.

4. Composition selon l'une quelconque des revendications 1 à 3, **caractérisée en ce que** le monomère (b) est choisi parmi le méthacrylate de N,N-diméthylaminoéthyle, l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyl-méthacrylamide, le N,N-diéthylaminopropyl-acrylamide et le N,N-diéthylaminopropyl-méthacrylamide, et est préférentiellement le méthacrylate de N,N-diméthylaminoéthyle.

5. Composition selon l'une quelconque des revendications 1 à 4, **caractérisée en ce que** le monomère (c) est un monomère tensioactif insaturé éthylénique copolymérisable obtenu par condensation d'un tensioactif non-ionique avec l'acide itaconique.

6. Composition selon l'une quelconque des revendications 1 à 5, **caractérisée en ce que** le terpolymère acrylique est constitué d'acrylates, d'amino(méth)acrylates et d'itaconate d'alkyle en C₁₀-C₃₀ polyoxyéthyléné à 20 moles d'oxyde d'éthylène.

7. Composition selon l'une quelconque des revendications 1 à 6, **caractérisée par le fait que** le sel de pyridinethione est choisi parmi les sels de calcium, de magnésium, de barium, de strontium, de zinc, de cadmium, d'étain et de zirconium, et préférentiellement le sel de zinc.

8. Composition selon l'une quelconque des revendications 1 à 7, **caractérisée par le fait que** le sel de pyridinethione est présent à raison de 0,001 à 10% en poids, de préférence de 0,01 à 5% en poids par rapport au poids total de la composition.

9. Composition selon l'une quelconque des revendications 1 à 8, **caractérisée en ce qu'**elle contient en outre au moins un agent tensio-actif choisi parmi les agents tensio-actifs anioniques, amphotères, non-ioniques, cationiques et leurs mélanges.

10. Composition selon la revendication 9, **caractérisée par le fait que** les agents tensio-actifs anioniques sont choisis parmi les sels alcalins, les sels de magnésium, les sels d'ammonium, les sels d'amines ou les sels d'aminoalcools des composés suivants : les alkylsulfates, alkyléthersulfates, alkylamidoéthersulfates, alkylarylpolyéthersulfates, monoglycérides sulfates; les alkylsulfonates, alkylamides sulfonates, alkylaryl-sulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates; le radical alkyle ou acyle de ces différents composés étant constitué par une chaîne carbonée comportant de 8 à 30 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 30 atomes de carbone; les acides d'alkyl D-galactoside uroniques et leurs sels, les acides alkyl ou alkylaryl éthers carboxyliques polyoxyalkylénés ou leurs sels, les acides alkylamido éthers carboxyliques polyoxyalkylénés ou leurs sels.

11. Composition selon la revendication 9, **caractérisée par le fait que** les agents tensio-actifs non-ioniques sont choisis parmi les alcools ou les alkylphénols ou les acides gras polyéthoxylés, polyoxypropylénés ou polyglycérolés, à chaîne grasse comportant 8 à 30 atomes de carbone, le nombre de groupements oxyde d'éthylène, oxyde de propylène étant compris entre 2 et 50 et le nombre de groupements glycérol étant compris entre 2 et 30; les copolymères d'oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les dérivés amides ou carbamates de N-alkylglucamines, les aldobionamides et les oxydes d'amines.

12. Composition selon la revendication 9, **caractérisée par le fait que** les agents tensio-actifs amphotères sont choisis parmi les dérivés d'amines secondaires ou tertiaires aliphatiques, dans lesquels le radical aliphatique est une chaîne linéaire ou ramifiée comportant de 8 à 22 atomes de carbone et qui contient au moins un groupe anionique hydrosolubilisant, carboxylate, sulfonate, sulfate, phosphate, phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes

13. Composition selon la revendication 9, **caractérisée par le fait que** les agents tensio-actifs cationiques sont choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

14. Composition selon l'une quelconque des revendications 9 à 13, **caractérisée en ce que** l'agent tensio-actif est présent à raison de 0,01 % à 50% en poids par rapport au poids total de la composition.

15. Composition selon la revendication 14, **caractérisée en ce que** l'agent tensioactif est présent à raison d'au moins 4% en poids, de préférence de 5 à 50% en poids, et encore plus préférentiellement de 8 à 35% en poids par rapport au poids total de la composition.

16. Composition selon l'une quelconque des revendications 1 à 15, **caractérisée en ce que** l'agent de conditionnement insoluble est choisi parmi les céramides, les cires, les silicones insolubles et les huiles non siliconées.

17. Composition selon la revendication 16, **caractérisée par le fait que** les céramides sont choisis parmi les céramides et/ou les glycocéramides et/ou les pseudocéramides et/ou les néocéramides, naturels ou synthétiques.

18. Composition selon la revendication 16, **caractérisée par le fait que** les cires sont choisies parmi les cires animales, les cires végétales, les cires minérales et les cires synthétiques.

19. Composition selon la revendication 16, **caractérisée par le fait que** les silicones insolubles sont choisies parmi les silicones volatiles et les silicones non-volatiles et notamment parmi:
(i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes organomodifiés ;
(vii) les copolymères blocs ayant un bloc linéaire polysiloxane-polyalkylène comme unité répétitive ;
(viii) les polymères siliconés greffés, à squelette organique non siliconé;
(ix) les polymères siliconés greffés, à squelette polysiloxanique greffé par des monomères organiques non siliconés;
(x) et leurs mélanges.

20. Composition selon la revendication 16, **caractérisée en ce que** les huiles non siliconées sont choisies parmi les huiles de synthèse, les huiles minérales, végétales ou animales, les alcools gras insaturés et les esters d'acides gras et de mono-ou polyalcools inférieurs en C₂-C₄.

21. Composition selon l'une quelconque des revendications 1 à 20, **caractérisée par le fait que** l'agent de conditionnement insoluble est utilisé dans des proportions de 0,001 à 60% en poids et de préférence de 0,01 à 40% en poids par rapport au poids total de la composition.

22. Composition selon l'une quelconque des revendications 1 à 21, **caractérisée en ce qu'**elle présente un pH compris entre 3 et 12, et plus particulièrement entre 4 et 8.

23. Composition selon l'une quelconque des revendications 1 à 22, **caractérisée par le fait que** le milieu cosmétiquement acceptable est constitué par de l'eau ou par un ou plusieurs solvants ou par un mélange d'eau et d'au moins un solvant cosmétiquement acceptable choisi parmi les alcools inférieurs, les alkylèneglycols et les éthers de polyol.

24. Composition selon l'une quelconque des revendications 1 à 23, **caractérisée par le fait qu'**elle contient en outre au moins un polymère cationique choisi parmi :
- les protéines ou hydrolysats de protéines, en particulier les hydrolysats de collagène portant des groupements triéthylammonium, les hydrolysats de collagène portant des groupements chlorure de triméthylammonium et de triméthylstéarylammonium, les hydrolysats de protéines portant sur la chaîne polypeptidique des groupements ammonium quaternaire comportant au moins un radical alkyle ayant de 1 à 18 atomes de carbone, les protéines végétales quaternisées telles que les protéines de blé, de maïs ou de soja;
- les polymères du type polyamine, polyaminoamide, polyammonium quaternaire, en particulier
i) les copolymères vinylpyrrolidone-acrylate ou méthacrylate de dialkylaminoalkyle quaternisés ou non,
ii) les dérivés d'éthers de cellulose comportant des groupements ammonium quaternaires,
iii) les dérivés de cellulose cationiques tels que les copolymères de cellulose ou les dérivés de cellulose greffés avec un monomère hydrosoluble d'ammonium quaternaire,
iv) les polysaccharides et notamment gommes de guar cationiques,
v) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
vi) les polyaminoamides solubles dans l'eau préparés en particulier par polycondensation d'un composé acide avec une polyamine,
vii) les dérivés de polyaminoamides résultant de la condensation de polyalcoylène polyamines avec des acides polycarboxyliques suivie d'une alcoylation par des agents bifonctionnels,
viii) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
ix) les cyclopolymères de méthyl diallyl amine ou de diméthyl diallyl ammonium,
x) les polymères de diammonium quaternaire,
xi) les polymères de polyammonium quaternaire,
xii) les homopolymères ou copolymères dérivés des acides acrylique ou méthacrylique et comportant des motifs CH₂-CHRₐ-CO-O-A₁-NRₑR_{f} CH₂-CHRₐ-CO-O-A₁-N⁺R_{b}R_{c}R_{d}, X⁻ et/ou CH₂-CHRₐ-CO-NH-A₁-N⁺R_{b}R_{c}R_{d}, X⁻
dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
xiii) les polymères quaternaires de vinylpyrrolidone et de vinylimidazole,
xiv) les polyamines référencées sous le nom de « POLYETHYLENEGLYCOL TALLOW POLYAMINE » dans le dictionnaire CTFA,
xv) les polymères réticulés de sels de méthacryloyloxyéthyl triméthylammonium,
- les polyalkylèneimines, les polymères contenant des motifs vinylpyridine ou vinylpyridinium, les condensats de polyamines et d'épichlorhydrine, les polyuréylènes quaternaires et les dérivés de la chitine.

25. Composition selon la revendication 24, **caractérisée par le fait qu'**elle contient un polymère cationique dans des proportions comprises entre 0,001 et 20% en poids et de préférence entre 0,05 et 5% en poids par rapport au poids total de la composition.

26. Composition selon l'une quelconque des revendications 1 à 25, **caractérisée par le fait qu'**elle contient en outre au moins un adjuvant cosmétiquement acceptable choisi parmi les parfums, les conservateurs, les séquestrants, les humectants, les sucres, les polymères amphotères, le menthol, les filtres, les dérivés de nicotinates, les agents antichute des cheveux, les stabilisateurs de mousse, les agents propulseurs, les colorants, les vitamines ou provitamines et les agents acidifiants ou alcalinisants.

27. Utilisation cosmétique comme shampooing, lotion ou après-shampooing d'une composition telle que définie dans l'une quelconque des revendications 1 à 26.

28. Procédé cosmétique de lavage des matières kératiniques, **caractérisé par le fait que** l'on applique sur ces matières au moins une composition telle que définie dans l'une quelconque des revendications 1 à 26, et facultativement, après un temps de pose facultatif, on les rince à l'eau.

## Claims

1. Antidandruff composition for treating the hair and the scalp, comprising at least one pyridinethione salt and at least one insoluble conditioner in a cosmetically acceptable medium, **characterized in that** it also comprises at least one acrylic terpolymer consisting of:
- from 5% to 80% by weight, preferably from 15% to 70% by weight and more preferably from 40% to 70% by weight of an acrylate monomer (a) chosen from a C₁-C₆ alkyl acrylate and a C₁-C₆ alkyl methacrylate;
- from 5% to 80% by weight, preferably from 10% to 70% by weight and more preferably from 20% to 60% by weight, of a monomer (b) chosen from a heterocyclic vinyl compound containing at least one nitrogen or sulfur atom, a (meth)acrylamide, a mono- or di (C₁-C₄) alkylamino (C₁-C₄) alkyl (meth) acrylate and a mono- or di (C₁-C₄) alkylamino (C₁-C₄) alkyl (meth) acrylamide;
- from 0.1% to 30% by weight, preferably from 0.1% to 10% by weight, of a monomer (c) chosen from:
a urethane produced by reaction between a monoethylenic unsaturated isocyanate and a nonionic surfactant comprising a block copolymer of 1,2-butylene oxide and of ethylene oxide with a C₁₋₄ alkoxy end;
a copolymerizable ethylenic unsaturated surfactant monomer obtained by condensation of a nonionic surfactant with an α,β-ethylenic unsaturated carboxylic acid or its anhydride;
a surfactant monomer chosen from the products of reaction of the type such as a urea of a monoethylenic unsaturated monoisocyanate with a nonionic surfactant containing an amine function;
a (meth) allyl ether of formula CH₂=CR₁CH₂OAₘBₙAₚR₂ in which R₁ denotes a hydrogen atom or a methyl group, A denotes a propylenoxy or butylenoxy group, B denotes ethylenoxy, n is equal to zero or denotes an integer less than or equal to 200, m and p denote zero or an integer less than n and R₂ is a hydrophobic group of at least 8 carbon atoms; and
a nonionic monomer of urethane type produced by reaction of a monohydric nonionic surfactant with a monoethylenic unsaturated isocyanate;
- and optionally at least one crosslinking monomer;
the weight percentages of monomers being based on the total weight of the monomers constituting the terpolymer.

2. Composition according to Claim 1, **characterized in that** the terpolymer is present in a proportion of from 0.01% to 20% by weight of active material, preferably 0.1% to 10% by weight, relative to the total weight of the composition.

3. Composition according to Claim 1 or 2, **characterized in that** the monomer (a) is chosen from C₂-C₆ alkyl acrylates and is preferably ethyl acrylate.

4. Composition according to any one of Claims 1 to 3, **characterized in that** the monomer (b) is chosen from N,N-dimethylaminoethyl methacrylate, N,N-diethylaminoethyl acrylate, N,N-diethylaminoethyl methacrylate, N-t-butylaminoethyl acrylate, N-t-butylaminoethyl methacrylate, N,N-dimethylaminopropyl-acrylamide, N,N-dimethylaminopropylmethacrylamide, N,N-diethylaminopropylacrylamide and N,N-diethylaminopropylmethacrylamide and is preferably N,N-dimethylaminoethyl methacrylate.

5. Composition according to any one of Claims 1 to 4, **characterized in that** the monomer (c) is a copolymerizable ethylenic unsaturated surfactant monomer obtained by condensing a nonionic surfactant with itaconic acid.

6. Composition according to any one of Claims 1 to 5, **characterized in that** the acrylic terpolymer consists of acrylates, amino (meth)acrylates and C₁₀-C₃₀ alkyl itaconate, polyoxyethylenated with 20 mol of ethylene oxide.

7. Composition according to any one of Claims 1 to 6, **characterized in that** the pyridinethione salt is chosen from calcium, magnesium, barium, strontium, zinc, cadmium, tin and zirconium salts and preferably the zinc salt.

8. Composition according to any one of Claims 1 to 7, **characterized in that** the pyridinethione salt is present in a proportion of from 0.001% to 10% by weight, preferably from 0.01% to 5% by weight, relative to the total weight of the composition.

9. Composition according to any one of Claims 1 to 8, **characterized in that** it also contains at least one surfactant chosen from anionic, amphoteric, nonionic and cationic surfactants and mixtures thereof.

10. Composition according to Claim 9, **characterized in that** the anionic surfactants are chosen from alkaline salts, magnesium salts, ammonium salts, amine salts or amino alcohol salts of the following compounds: alkyl sulfates, alkyl ether sulfates, alkylamido ether sulfates, alkylaryl polyether sulfates, monoglyceride sulfates; alkyl sulfonates, alkylamide sulfonates, alkylaryl sulfonates, olefin sulfonates, paraffin sulfonates; alkyl sulfosuccinates, alkyl ether sulfosuccinates, alkylamide sulfosuccinates; alkyl sulfosuccinamates; alkyl sulfoacetates; alkyl phosphates, alkyl ether phosphates; acyl sarcosinates, acyl isethionates and N-acyl taurates; the alkyl or acyl radical in these various compounds generally consisting of a carbon-based chain containing from 8 to 30 carbon atoms; the fatty acid salts of oleic, ricinoleic, palmitic and stearic acid salts; coconut oil acid or hydrogenated coconut oil acid; acyl lactylates, in which the acyl radical contains from 8 to 30 carbon atoms; alkyl D-galactosiduronic acids and salts thereof, polyoxyalkylenated alkyl or alkylaryl ether carboxylic acids or salts thereof, and polyoxyalkylenated alkylamido ether carboxylic acids or salts thereof.

11. Composition according to Claim 9, **characterized in that** the nonionic surfactants are chosen from polyethoxylated, polypropoxylated or polyglycerolated fatty acids or alkylphenols or alcohols, with a fatty chain containing 8 to 30 carbon atoms, the number of ethylene oxide or propylene oxide groups being between 2 and 50 and the number of glycerol groups being between 2 and 30; the copolymers of ethylene oxide and propylene oxide; condensates of ethylene oxide and of propylene oxide with fatty alcohols; polyethoxylated fatty amides; polyglycerolated fatty amides ; polyethoxylated fatty amines; oxyethylenated fatty acid esters of sorbitan; fatty acid esters of sucrose or of polyethylene glycol; alkylpolyglycosides; carbamate or amide derivatives of N-alkylglucamines, aldobionamides, and amine oxides.

12. Composition according to Claim 9, **characterized in that** the amphoteric surfactants are chosen from secondary or tertiary aliphatic amine derivatives, in which the aliphatic radical is a linear or branched chain containing 8 to 22 carbon atoms and which contains at least one carboxylate, sulfonate, sulfate, phosphate or phosphonate water-solubilizing anionic group; (C₈-C₂₀) alkylbetaines, sulfobetaines, (C₈-C₂₀) alkylamido (C₁-C₆) alkylbetaines or (C₈-C₂₀)alkylamido(C₁-C₆)alkylsulfobetaines.

13. Composition according to Claim 9, **characterized in that** the cationic surfactants are chosen from optionally polyoxyalkylenated primary, secondary or tertiary fatty amine salts; quaternary ammonium salts; imidazoline derivatives; and amine oxides of cationic nature.

14. Composition according to any one of Claims 9 to 13, **characterized in that** the surfactant is present in a proportion of from 0.01% to 50% by weight relative to the total weight of the composition.

15. Composition according to Claim 14, **characterized in that** the surfactant is present in a proportion of at least 4% by weight, preferably from 5% to 50% by weight and even more preferably from 8% to 35% by weight, relative to the total weight of the composition.

16. Composition according to any one of Claims 1 to 15, **characterized in that** the insoluble conditioner is chosen from ceramides, waxes, insoluble silicones and non-silicone oils.

17. Composition according to Claim 16, **characterized in that** the ceramides are chosen from natural or synthetic ceramides and/or glycoceramides and/or pseudoceramides and/or neoceramides.

18. Composition according to Claim 16, **characterized in that** the waxes are chosen from animal waxes, plant waxes, mineral waxes and synthetic waxes.

19. Composition according to Claim 16, **characterized in that** the insoluble silicones are chosen from volatile silicones and non-volatile silicones and in particular from:
(i) polyalkylsiloxanes;
(ii) polyarylsiloxanes;
(iii) polyalkylarylsiloxanes;
(iv) silicone gums;
(v) silicone resins;
(vi) organomodified polyorganosiloxanes;
(vii) block copolymers containing a linear polysiloxane-polyalkylene block as repeating unit;
(viii)grafted silicone polymers, containing a non-silicone organic skeleton;
(xi) grafted silicone polymers, containing a polysiloxane skeleton grafted with non-silicone organic monomers;
(x) and mixtures thereof.

20. Composition according to Claim 16, **characterized in that** the non-silicone oils are chosen from synthetic oils, mineral oils, plant oils and animal oils, unsaturated fatty alcohols and fatty acid esters of lower C₂-C₄ mono- or polyalcohols.

21. Composition according to any one of Claims 1 to 20, **characterized in that** the insoluble conditioner is used in proportions of from 0.001% to 60% by weight, and preferably from 0.01% to 40% by weight, relative to the total weight of the composition.

22. Composition according to any one of Claims 1 to 21, **characterized in that** it has a pH of between 3 and 12 and more particularly between 4 and 8.

23. Composition according to any one of Claims 1 to 22, **characterized in that** the cosmetically acceptable medium consists of water or of one or more solvents or of a mixture of water and of at least one cosmetically acceptable solvent chosen from lower alcohols, alkylene glycols and polyol ethers.

24. Composition according to any one of Claims 1 to 23, **characterized in that** it also contains at least one cationic polymer chosen from:
- proteins or protein hydrolysates, in particular collagen hydrolysates bearing triethylammonium groups, collagen hydrolysates bearing trimethylammonium chloride and trimethylstearylammonium chloride groups, protein hydrolysates bearing on the polypeptide chain quaternary ammonium groups comprising at least one alkyl radical containing from 1 to 18 carbon atoms, and quaternized plant proteins such as wheat, corn or soybean proteins;
- polymers of the polyamine, polyaminoamide or polyquaternary ammonium type, in particular
i) Quaternized or non-quaternized vinyl-pyrrolidone/dialkylaminoalkyl acrylate or methacrylate copolymers,
ii) cellulose ether derivatives comprising quaternary ammonium groups,
iii) cationic cellulose derivatives such as cellulose copolymers or cellulose derivatives grafted with a water-soluble quaternary ammonium monomer,
iv) polysaccharides and in particular cationic guar gums,
v) polymers consisting of piperazinyl units and of divalent alkylene or hydroxyalkylene radicals containing straight or branched chains, optionally interrupted with oxygen, sulfur or nitrogen atoms or with aromatic or heterocyclic rings, as well as the oxidation and/or quaternization products of these polymers,
vi) water-soluble polyaminoamides prepared in particular by polycondensation of an acidic compound with a polyamine,
vii) polyaminoamide derivatives resulting from the condensation of polyalkylene polyamines with polycarboxylic acids followed by an alkylation with bifunctional agents,
viii) polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms,
ix) cyclopolymers of methyldiallylamine or of dimethyldiallylammonium,
x) the diquaternary ammonium polymers,
xi) the polyquaternary ammonium polymers,
xii) homopolymers or copolymers derived from acrylic or methacrylic acids and comprising CH₂-CHRₐ-CO-O-A₁-NRₑR_{f}, CH₂-CHRₐ-CO-O-A₁-N⁺R_{b}R_{c}R_{d}, X⁻ and/or CH₂-CHRₐ-CO-NH-A₁-N⁺R_{b}R_{c}R_{d}, X⁻ units,
in which the groups Rₐ independently denote H or CH₃, the groups A₁ independently denote a linear or branched alkyl group of 1 to 6 carbon atoms or a hydroxyalkyl group of 1 to 4 carbon atoms, the groups R_{b}, R_{c} and R_{d}, which may be identical or different, independently denote an alkyl group of 1 to 18 carbon atoms or a benzyl radical, the groups Rₑ and R_{f} represent a hydrogen atom or an alkyl group of 1 to 6 carbon atoms, X⁻ denotes an anion, for example methosulfate or halide, such as chloride or bromide,
xiii) quaternary polymers of vinylpyrrolidone and of vinylimidazole,
xiv) the polyamines referred to under the name "Polyethylene Glycol Tallow Polyamine" in the CTFA dictionary,
xv) crosslinked methacryloyloxyethyltrimethylammonium salt polymers,
- polyalkyleneimines, polymers containing vinylpyridine or vinylpyridinium units, condensates of polyamines and of epichlorohydrin, polyquaternary ureylenes and chitin derivatives.

25. Composition according to Claim 24, **characterized in that** it contains a cationic polymer in proportions of between 0.001% and 20% by weight and preferably between 0.05% and 5% by weight relative to the total weight of the composition.

26. Composition according to any one of Claims 1 to 25, **characterized in that** it also contains at least one cosmetically acceptable adjuvant chosen from fragrances, preserving agents, sequestering agents, wetting agents, sugars, amphoteric polymers, menthol, screening agents, nicotinate derivatives, agents for preventing hair loss, foam stabilizers, propellants, dyes, vitamins or provitamins and acidifying or basifying agents.

27. Cosmetic use, as a shampoo, lotion or conditioner, of a composition as defined in any one of Claims 1 to 26.

28. Cosmetic process for washing keratin materials, **characterized in that** at least one composition as defined in any one of Claims 1 to 26 is applied to these materials and, after optionally leaving the composition on the hair for a period of time, the hair is optionally rinsed with water

## Patentansprüche

1. Zusammensetzung für die Antischuppenbehandlung der Haare und der Kopfhaut, die in einem kosmetisch akzeptablen Medium mindestens ein Pyridinthionsalz und mindestens ein unlösliches Konditioniermittel enthält, **dadurch gekennzeichnet, dass** sie außerdem mindestens ein Acrylterpolymer enthält, das besteht aus:
- 5 bis 80 Gew.-%, vorzugsweise 15 bis 70 Gew.-% und noch bevorzugter 40 bis 70 Gew.-% eines Acrylatmonomers (a), das unter C₁₋₆-Alkylacrylaten und C₁₋₆-Alkylmethacrylaten ausgewählt wird;
- 5 bis 80 Gew.-%, vorzugsweise 10 bis 70 Gew.-% und noch bevorzugter 20 bis 60 Gew.-% eines Monomers (b), das unter den heterocyclischen Vinylverbindungen, die mindestens ein Stickstoffatom oder Schwefelatom enthalten, (Meth)acrylamid, den Mono-C₁₋₄-alkylamino-C₁₋₄-alkyl(meth)acrylaten und Di-C₁₋₄-alkylamino-C₁₋₄-alkyl(meth)acrylaten und den Mono-C₁₋₄-alkylamino-C₁₋₄-alkyl(meth)acrylamiden und Di-C₁₋₄-Alkylamino-C₁₋₄-alkyl(meth)acrylamiden ausgewählt wird;
- 0,1 bis 30 Gew.-%, vorzugsweise 0,1 bis 10 Gew.-% eines Monomers (c), das ausgewählt wird unter:
den Urethanen, die durch die Umsetzung eines monoethylenisch ungesättigten Isocyanats mit einem nichtionischen grenzflächenaktiven Stoff, der ein sequentielles Copolymer aus 1,2-Butylenoxid und Ethylenoxid mit C₁₋₄-Alkoxyend-gruppe einschließt, erzeugt werden;
den copolymerisierbaren ethylenisch ungesättigten grenzflächenaktiven Monomeren, die durch die Kondensation eines nichtionischen grenzflächenaktiven Stoffs mit einer α,β-ethylenisch ungesättigten Carbonsäure oder deren Anhydrid erhalten werden;
den grenzflächenaktiven Monomeren, die unter den Reaktionsprodukten vom Harnstofftyp der Umsetzung eines monoethylenisch ungesättigten Monoisocyanats mit einem nichtionischen grenzflächenaktiven Stoff, der eine funktionelle A-mingruppe aufweist, ausgewählt werden;
den (Meth)allylethern der Formel CH₂=CR₁CH₂OAₘBₙAₚR₂, worin R₁ ein Wasserstoffatom oder eine Methylgruppe bedeutet, A eine Propylenoxy- oder Butylenoxygruppe ist, B Ethylenoxy bedeutet, n gleich Null ist oder eine ganze Zahl kleiner als oder gleich 200 bedeutet, m und p Null oder eine ganze Zahl kleiner als n bedeuten und R₂ eine hydrophobe Gruppe mit mindestens 8 Kohlenstoffatomen ist; und
den nichtionischen Monomeren vom Urethantyp, die durch die Umsetzung eines nichtionischen grenzflächenaktiven Stoffs mit einem reaktiven Wasserstoffatom mit einem monoethylenisch ungesättigten Isocyanat erzeugt werden;
- und gegebenenfalls mindestens einem Vernetzungsmonomer;
wobei die Gewichtsprozentangaben zu den Monomeren auf das Gesamtgewicht der Monomere bezogen sind, die das Terpolymer bilden.

2. Zusammensetzung nach Anspruch 1, **dadurch gekennzeichnet, dass** das Terpolymer in einem Anteil von 0,01 bis 20 Gew.-% wirksame Substanz und vorzugsweise 0,1 bis 10 Gew.-% wirksame Substanz, bezogen auf das Gesamtgewicht der Zusammensetzung, enthalten ist.

3. Zusammensetzung nach Anspruch 1 oder 2, **dadurch gekennzeichnet, dass** das Monomer (a) unter den C₂₋₆-Alkylacrylaten ausgewählt wird und vorzugsweise Ethylacrylat ist.

4. Zusammensetzung nach einem der Ansprüche 1 bis 3, **dadurch gekennzeichnet, dass** das Monomer (b) unter N,N-Dimethylaminoethylmethacrylat, N,N-Diethylaminoethylacrylat, N,N-Diethylaminoethylmethacrylat, N-*tert*.-Butylaminoethylacrylat, N-*tert.-*Butylaminoethylmethacrylat, N,N-Dimethylaminopropylacrylamid, N,N-Dimethylaminopropylmethacrylamid, N,N-Diethylaminopropylacrylamid und N,N-Diethylaminopropylmethacrylamid ausgewählt wird und dass es sich bei dem Monomer (b) vorzugsweise um N,N-Dimethylaminoethylmethacrylat handelt.

5. Zusammensetzung nach einem der Ansprüche 1 bis 4, **dadurch gekennzeichnet, dass** das Monomer (c) ein copolymerisierbares, ethylenisch ungesättigtes grenzflächenaktives Monomer ist, das durch die Kondensation eines nichtionischen grenzflächenaktiven Stoffs mit Itaconsäure erhalten wird.

6. Zusammensetzung nach einem der Ansprüche 1 bis 5, **dadurch gekennzeichnet, dass** das Acrylterpolymer aus Acrylaten, Amino-(meth)acrylaten und C₁₀₋₃₀-Alkylitaconat, das mit 20 mol Ethylenoxid polyethoxyliert ist, besteht.

7. Zusammensetzung nach einem der Ansprüche 1 bis 6, **dadurch gekennzeichnet, dass** das Pyridinthionsalz unter dem Calciumsalz, dem Magnesiumsalz, dem Bariumsalz, dem Strontiumsalz, dem Zinksalz, dem Cadmiumsalz, dem Zinnsalz und dem Zirconiumsalz ausgewählt wird und dass es sich vorzugsweise um das Zinksalz handelt.

8. Zusammensetzung nach einem der Ansprüche 1 bis 7, **dadurch gekennzeichnet, dass** das Pyridinthionsalz in einem Anteil von 0,001 bis 10 Gew.-%, vorzugsweise 0,01 bis 5 Gew.-%, bezogen auf das Gesamtgewicht der Zusammensetzung, enthalten ist.

9. Zusammensetzung nach einem der Ansprüche 1 bis 8, **dadurch gekennzeichnet, dass** sie außerdem mindestens einen grenzflächenaktiven Stoff enthält, der unter den anionischen, den amphoteren, den nichtionischen, den kationischen grenzflächenaktiven Stoffen und deren Gemischen ausgewählt wird.

10. Zusammensetzung nach Anspruch 9, **dadurch gekennzeichnet, dass** die anionischen grenzflächenaktiven Stoffe ausgewählt werden unter den Alkalisalzen, den Magnesiumsalzen, den Ammoniumsalzen, den Aminsalzen oder den Aminoalkoholsalzen der folgenden Verbindungen: der Alkylsulfate, Alkylethersulfate, Alkylamidoethersulfate, Alkylarylpolyethersulfate, Monoglyceridsulfate; der Alkylsulfonate, Alkylamidsulfonate, Alkylarylsulfonate, Olefinsulfonate, Paraffinsulfonate; der Alkylsulfosuccinate, der Alkylethersulfosuccinate, der Alkylamidsulfosuccinate; der Alkylsulfosuccinamate; der Alkylsulfoacetate; der Alkylphosphate, Alkyletherphosphate; der Acylsarconsinate, der Acylisethionate, N-Acyltaurate; wobei der Alkylrest oder Acylrest dieser verschiedenen Verbindungen aus einer Kohlenstoffkette besteht, die 8 bis 30 Kohlenstoffatome aufweist; den Fettsäuresalzen der Ölsäure, Ricinolsäure, Palmitinsäure, Stearinsäure; der Säuren von Koprahöl oder hydriertem Koprahöl; den Acyllactylaten, deren Acylrest 8 bis 30 Kohlenstoffatome aufweist; den Alkyl-D-galactosiduronsäuren und deren Salzen, den polyalkoxylierten Alkyl- oder Alkylarylethercarbonsäuren und deren Salzen, den polyalkoxylierten Alkylamidoethercarbonsäuren und deren Salzen.

11. Zusammensetzung nach Anspruch 9, **dadurch gekennzeichnet, dass** die nichtionischen grenzflächenaktiven Stoffe ausgewählt werden unter den polyethoxylierten, polypropoxylierten oder mehrfach mit Glycerin veretherten Alkoholen oder Alkylphenolen oder Fettsäuren, mit einer Fettkette, die 8 bis 30 Kohlenstoffatome aufweist, wobei die Anzahl der Ethylenoxidgruppen, der Propylenoxidgruppen im Bereich von 2 bis 50 liegt und die Anzahl der Glyceringruppen im Bereich von 2 bis 30 liegt; Copolymeren aus Ethylenoxid und Propylenoxid; den Kondensaten von Ethylenoxid und Propylenoxid mit Fettalkoholen; den polyethoxylierten Fettamiden; den mehrfach mit Glycerin veretherten Fettamiden; den polyethoxylierten Fettaminen; den ethoxylierten Estern aus Fettsäuren und Sorbitan; den Estern aus Fettsäuren und Saccharose oder Polyethylenglycol; den Alkylpolyglycosiden; den Amid- oder Carbamatderivaten von N-Alkylglucaminen, den Aldobionamiden und den Aminoxiden.

12. Zusammensetzung nach Anspruch 9, **dadurch gekennzeichnet, dass** die amphoteren grenzflächenaktiven Stoffe unter den Derivaten sekundärer oder tertiärer aliphatischer Amine, in denen der aliphatische Rest eine geradkettige oder verzweigte Kette ist, die 8 bis 22 Kohlenstoffatome enthält und die mindestens eine wasserlöslich machende anionische Carboxylat-, Sulfonat-, Sulfat-, Phosphat-, Phosphonatgruppe enthält; den C₈₋₂₀-Alkylbetainen, den Sulfobetainen, den C₈₋₂₀-Alkyl-C₁₋₆-amidoalkylbetainen und den C₈₋₂₀-Alkyl-C₁₋₆-amidoalkylsulfobetainen ausgewählt wird.

13. Zusammensetzung nach Anspruch 9, **dadurch gekennzeichnet, dass** die kationischen grenzflächenaktiven Stoffe unter den Salzen von primären, sekundären oder tertiären Fettaminen, die gegebenenfalls polyalkoxyliert sind; den quartären Ammoniumsalzen; den Imidazolinderivaten und den Aminoxiden mit kationischem Charakter ausgewählt werden.

14. Zusammensetzung nach einem der Ansprüche 9 bis 13, **dadurch gekennzeichnet, dass** der grenzflächenaktive Stoff in einem Anteil im Bereich von 0,01 bis 50 Gew.-%, bezogen auf das Gesamtgewicht der Zusammensetzung, enthalten ist.

15. Zusammensetzung nach Anspruch 14, **dadurch gekennzeichnet, dass** der grenzflächenaktive Stoff in einem Anteil von mindestens 4 Gew.-%, vorzugsweise 5 bis 50 Gew.-%, noch bevorzugter 8 bis 35 Gew.-%, bezogen auf das Gesamtgewicht der Zusammensetzung, enthalten ist.

16. Zusammensetzung nach einem der Ansprüche 1 bis 15, **dadurch gekennzeichnet, dass** das unlösliche Konditioniermittel unter den Ceramiden, den Wachsen, den unlöslichen Siliconen und den nicht siliconierten Ölen ausgewählt wird.

17. Zusammensetzung nach Anspruch 16, **dadurch gekennzeichnet, dass** die Ceramide unter den Ceramiden und/oder den Glycoceramiden und/oder den Pseudoceramiden und/oder den Neoceramiden, die natürlich oder synthetisch sind, ausgewählt werden.

18. Zusammensetzung nach Anspruch 16, **dadurch gekennzeichnet, dass** die Wachse unter den tierischen Wachsen, den pflanzlichen Wachsen, den Mineralwachsen und den synthetischen Wachsen ausgewählt werden.

19. Zusammensetzung nach Anspruch 16, **dadurch gekennzeichnet, dass** die unlöslichen Silicone unter den flüchtigen Siliconen und den nichtflüchtigen Siliconen und insbesondere unter
(i) den Polyalkylsiloxanen;
(ii) den Polyarylsiloxanen;
(iii) den Polyalkylarylsiloxanen;
(iv) den Silicongummis;
(v) den Siliconharzen;
(vi) den organisch modifizierten Polyorganosiloxanen;
(vii) den Blockcopolymeren, die einen linearen Polysiloxanpolyalkylenblock als wiederkehrende Einheit aufweisen;
(viii) den gepfropften Siliconpolymeren mit nicht siliconiertem organischem Rückgrat;
(ix) den gepfropften Siliconpolymeren mit gepfropftem Polysiloxanrückgrat, auf das nicht siliconierte organische Monomere gepfropft sind;
(x) und deren Gemischen
ausgewählt werden.

20. Zusammensetzung nach Anspruch 16, **dadurch gekennzeichnet, dass** die nicht siliconierten Öle unter den Syntheseölen, den Mineralölen, den pflanzlichen oder tierischen Ölen, den ungesättigten Fettalkoholen und den Estern aus Fettsäuren und niederen einwertigen Alkoholen und Polyolen mit zwei bis vier Kohlenstoffatomen ausgewählt werden.

21. Zusammensetzung nach einem der Ansprüche 1 bis 20, **dadurch gekennzeichnet, dass** das unlösliche Konditioniermittel in Anteilen von 0,001 bis 60 Gew.-% und vorzugsweise 0,01 bis 40 Gew.-%, bezogen auf das Gesamtgewicht der Zusammensetzung, verwendet wird.

22. Zusammensetzung nach einem der Ansprüche 1 bis 21, **dadurch gekennzeichnet, dass** sie einen pH-Wert aufweist, der im Bereich von 3 bis 12 und vor allem 4 bis 8 liegt.

23. Zusammensetzung nach einem der Ansprüche 1 bis 22, **dadurch gekennzeichnet, dass** das kosmetisch akzeptable Medium aus Wasser oder aus einem oder mehreren Lösemitteln oder aus einem Gemisch aus Wasser und mindestens einem kosmetisch akzeptablen Lösemittel besteht, das unter den niederen Alkoholen, den Alkylenglycolen und den Polyolethern ausgewählt wird.

24. Zusammensetzung nach einem der Ansprüche 1 bis 23, **dadurch gekennzeichnet, dass** sie außerdem mindestens ein kationisches Polymer enthält, das ausgewählt wird unter:
- den Proteinen oder Proteinhydrolysaten, vor allem den Kollagenhydrolysaten, die Triethylammoniumgruppen tragen, den Kollagenhydrolysaten, die Trimethylammoniumchlorid- und Trimethylstearylammoniumchloridgruppen tragen, den Proteinhydrolysaten, die auf der Polypeptidkette quartäre Ammoniumgruppen tragen, die mindestens einen Alkylrest aufweisen, der 1 bis 18 Kohlenstoffatome hat, den quaternisierten pflanzlichen Proteinen, wie den Proteinen von Weizen, Mais oder Soja;
- den Polymeren vom Polyamintyp, Polyaminoamidtyp, vom quartären Polyammoniumtyp, vor allem
i) den Copolymeren aus Vinylpyrrolidon und quaternisiertem oder nicht quaternisiertem Dialkylaminoalkylacrylat oder -methacrylat,
ii) den Etherderivaten von Cellulose, die quartäre Ammoniumgruppen aufweisen,
iii) den kationischen Cellulosederivaten, wie den Cellulosecopolymeren oder den Cellulosederivaten, die mit einem wasserlöslichen quartären Ammoniummonomer gepfropft sind,
iv) den kationischen Polysacchariden und insbesondere den kationischen Guargummen,
v) den Polymeren, die aus Piperazinyleinheiten und zweiwertigen Alkylen- oder Hydroxyalkylenresten bestehen, mit geraden oder verzweigten Ketten, die gegebenenfalls durch Sauerstoff-, Schwefel- oder Stickstoffatome oder durch aromatische oder heterocyclische Ringe unterbrochen sind, sowie den Oxidationsprodukten und/oder Quaternisierungsprodukten dieser Polymere;
vi) den in Wasser löslichen Polyaminoamiden, die insbesondere durch Polykondensation einer sauren Verbindung mit einem Polyamin hergestellt werden,
vii) den Derivaten von Polyaminoamiden, die bei der Kondensation von Polyalkylenpolyaminen mit Polycarbonsäuren, auf die eine Alkylierung mit bifunktionellen Mitteln folgt, entstehen,
viii) den Polymeren, die erhalten werden durch die Umsetzung eines Polyalkylenpolyamins, das zwei primäre Amingruppen und mindestens eine sekundäre Amingruppe aufweist, mit einer Dicarbonsäure, die unter der Diglycolsäure und den gesättigten aliphatischen Dicarbonsäuren, die 3 bis 8 Kohlenstoffatome aufweisen, ausgewählt wird,
ix) den Methyldiallylamin- oder Dimethyldiallylammoniumcyclopolymeren,
x) den quartären Diammoniumpolymeren,
xi) den quartären Polyammoniumpolymeren,
xii) den Homopolymeren oder Copolymeren, die von Acrylsäure oder Methacrylsäure abgeleitet sind und die Einheiten CH₂-CHRₐ-CO-O-A₁-NRₑR_{f}, CH₂-CHRₐ-CO-O-A₁-N⁺R_{b}R_{c}R_{d}, X⁻ und/oder CH₂-CHRₐ-CO-NH-A₁-N⁺R_{b}R_{c}R_{d}, X- aufweisen, worin bedeuten: die Gruppen Rₐ unabhängig H oder CH₃, die Gruppen A₁ unabhängig eine geradkettige oder verzweigte Alkylgruppe mit 1 bis 6 Kohlenstoffatomen oder eine Hydroxyalkylgruppe mit 1 bis 4 Kohlenstoffatomen, die Gruppen R_{b}, R_{c}, R_{d}, gleich oder verschieden, unabhängig eine Alkylgruppe mit 1 bis 18 Kohlenstoffatomen oder einen Benzylrest, die Gruppen Rₑ und R_{f} ein Wasserstoffatom oder eine Alkylgruppe mit 1 bis 6 Kohlenstoffatomen, X- ein Anion, beispielsweise Methosulfat oder Halogenid, wie Chlorid oder Bromid,
xiii) den quartären Polymeren von Vinylpyrrolidon und Vinylimidazol,
xiv) den Polyaminen, die im CTFA-Lexikon unter der Bezeichnung "POLYETHYLENEGLYCOL TALLOW POLYAMINE" aufgeführt sind,
xv) den vernetzten Polymeren der Methacryloyloxyethyltrimethylammoniumsalze,
- den Polyalkyleniminen, den Polymeren, die Vinylpyridin- oder Vinylpyridiniumeinheiten enthalten, den Kondensaten von Polyaminen und Epichlorhydrin, den quartären Polyureylenen und den Chitinderivaten.

25. Zusammensetzung nach Anspruch 24, **dadurch gekennzeichnet, dass** sie ein kationisches Polymer in Anteilen enthält, die im Bereich von 0,001 bis 20 Gew.-% und vorzugsweise 0,05 bis 5 Gew.-%, bezogen auf das Gesamtgewicht der Zusammensetzung, liegen.

26. Zusammensetzung nach einem der Ansprüche 1 bis 25, **dadurch gekennzeichnet, dass** sie außerdem mindestens einen kosmetisch akzeptablen Zusatzstoff enthält, der unter den Parfüms, den Konservierungsmitteln, den Maskierungsmitteln, den Feuchthaltemitteln, den Zuckern, den amphoteren Polymeren, Menthol, den Filtern, den Nicotinatderivaten, den Mitteln gegen Haarausfall, den Schaumstabilisatoren, den Treibmitteln, den Färbemitteln, den Vitaminen oder Provitaminen und den Säuerungsmitteln oder alkalisch machenden Mitteln ausgewählt wird.

27. Kosmetische Verwendung einer Zusammensetzung, die wie in einem der Ansprüche 1 bis 26 definiert ist, als Haarwaschmittel, Lotion oder Haarspülung.

28. Kosmetisches Verfahren zum Waschen von Keratinmaterialien, **dadurch gekennzeichnet, dass** mindestens eine wie in einem der Ansprüche 1 bis 26 definierte Zusammensetzung auf diese Materialien aufgetragen wird und dass fakultativ nach einer fakultativen Einwirkzeit diese mit Wasser ausgespült werden.
